(19) **Europäisches Patentamt**
**European Patent Office**
**Office européen des brevets**

(11) **EP 4 671 753 A1**

(12) **EUROPEAN PATENT APPLICATION**
published in accordance with Art. 153(4) EPC

(43) Date of publication:
**31.12.2025 Bulletin 2026/01**

(51) International Patent Classification (IPC):
*G01N 33/00* (2006.01)     *G01D 18/00* (2006.01)
*G01D 21/02* (2006.01)

(21) Application number: **24861877.9**

(22) Date of filing: **28.08.2024**

(86) International application number:
**PCT/CN2024/115173**

(87) International publication number:
**WO 2025/051033 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.09.2023 CN 202311153451**

(71) Applicant: **AI Group GHG Management Center
Beijing 100010 (CN)**

(72) Inventors:
• **SUN, Qi
  Beijing 100010 (CN)**
• **SUN, Pu
  Beijing 100010 (CN)**

(74) Representative: **Gulde & Partner
Patent- und Rechtsanwaltskanzlei mbB
Berliner Freiheit 2
10785 Berlin (DE)**

(54) **VERIFICATION METHOD AND APPARATUS FOR GAS ANALYSIS SYSTEM, DEVICE, AND STORAGE MEDIUM**

(57)     Embodiments of the present application provide a method, apparatus, electric device, and computer-readable storage medium for verifying a gas analysis system, relating to the gas detection field. The method includes: verifying the gas analysis system based on a first confidence interval obtained by testing the gas analysis system in standard operating conditions, a second confidence interval obtained by testing the gas analysis system in simulated operating conditions, a third confidence interval obtained by testing the gas analysis system in ideal operating conditions, and a fourth confidence interval obtained by testing the gas analysis system in actual operating conditions. The method for verifying a gas analysis system according to the embodiments of the present application enables the determination of the validity of data measured by the gas analysis system.

EP 4 671 753 A1

```
┌─────────────────────────────────────────────────────────────┐
│ Test the gas analysis system using cylinder gases under a    │
│ plurality of standard operating conditions, respectively,    │ ⟩ S101
│ to determine a plurality of baseline linearity curves of the │
│ gas analysis system and a corresponding first confidence     │
│ interval.                                                     │
└─────────────────────────────────────────────────────────────┘
                            ↓
┌─────────────────────────────────────────────────────────────┐
│ Test the gas analysis system using simulated operating       │
│ condition data to determine a second confidence interval     │ ⟩ S102
│ for the plurality of baseline linearity curves.              │
└─────────────────────────────────────────────────────────────┘
                            ↓
┌─────────────────────────────────────────────────────────────┐
│ Test the gas analysis system using ideal operating           │
│ condition data of the object to be measured to determine a   │ ⟩ S103
│ third confidence interval for the plurality of baseline      │
│ linearity curves.                                            │
└─────────────────────────────────────────────────────────────┘
                            ↓
┌─────────────────────────────────────────────────────────────┐
│ Test the gas analysis system using first actual operating    │
│ condition data of the object to be measured to determine a   │ ⟩ S104
│ fourth confidence interval for the plurality of baseline     │
│ linearity curves.                                            │
└─────────────────────────────────────────────────────────────┘
                            ↓
┌─────────────────────────────────────────────────────────────┐
│ Verify the gas analysis system based on the first confidence │
│ interval, the second confidence interval, the third          │ ⟩ S105
│ confidence interval, and the fourth confidence interval.     │
└─────────────────────────────────────────────────────────────┘
```

**FIG 1**

## Description

### Technical Field

The present application relates to the technical field of gas detection, and specifically, to a method, apparatus, device, and computer-readable storage medium for verifying a gas analysis system.

### Background Art

In some industries requiring continuous and precise determination of data validity, the analysis system itself has a significant impact on data accuracy. How to determine the accuracy of data measured by the analysis system is an urgent problem to be solved.

### Summary of the Invention

Embodiments of the present application provide a method, apparatus, device, computer-readable storage medium, and computer program product for verifying a gas analysis system, used to solve the technical problem of being unable to determine the effectiveness of a gas analysis system.

According to one aspect of the embodiments of the present application, a method for verifying a gas analysis system is provided. The method includes:

Testing the gas analysis system using cylinder gases with a plurality of standard operating conditions, to determine a plurality of baseline linearity curves of the gas analysis system and a corresponding first confidence interval thereof;

Testing the gas analysis system using simulated operating condition data, to determine a second confidence interval for the plurality of baseline linearity curves, wherein the simulated operating condition data is obtained by simulation of a simulation system on actual operating conditions of an object to be measured;

Testing the gas analysis system using ideal operating condition data of the object to be measured, to determine a third confidence interval for the plurality of baseline linearity curves;

Testing the gas analysis system using first actual operating condition data of the object to be measured, to determine a fourth confidence interval for the plurality of baseline linearity curves; and

Verifying the gas analysis system based on the first confidence interval, the second confidence interval, the third confidence interval, and the fourth confidence interval.

In an alternative implementation, the verifying of the gas analysis system based on the first confidence interval, the second confidence interval, the third confidence interval, and the fourth confidence interval includes:

If entirety of the fourth confidence interval falls in a union range of the first confidence interval, the second confidence interval, and the third confidence interval, the gas analysis system passing verification; and

Determining the union range of the first confidence interval, the second confidence interval, and the third confidence interval as a linearity confidence interval.

In another alternative implementation, the verifying of the gas analysis system based on the first confidence interval, the second confidence interval, the third confidence interval, and the fourth confidence interval further includes:

If entirety of the fourth confidence interval falls in the union range of the first confidence interval, the second confidence interval, and the third confidence interval, measuring the gas analysis system using first operating condition data to determine a fifth confidence interval for the plurality of baseline linearity curves,

Wherein the first operating condition data is obtained from the simulated operating condition data and second actual operating condition data, and the second actual operating condition data is set as a portion of the first actual operating condition data corresponding to the remaining of the fourth confidence interval exposed out of the union range of the first confidence interval, the second confidence interval, and the third confidence interval; and

Determining the union of the first confidence interval, the second confidence interval, the third confidence interval, the fourth confidence interval, and the fifth confidence interval as the linearity confidence interval.

In yet another alternative implementation, the method further includes:

Measuring third actual operating condition data of the object to be measured using the gas analysis system to obtain measurement data;

Determining a confidence interval from the linearity confidence interval based on the measurement data;
Determining invalid data and valid data within the measurement data based on the determined confidence interval.

In yet another alternative implementation, said determining invalid data and valid data within the measurement data based on the determined confidence interval includes:

Determining first data within the measurement data that does not exceed the determined confidence interval as the valid data; and
Determining second data within the measurement data that exceeds the determined confidence interval at a first timing or within a first time period as the invalid data.

In yet another alternative implementation, the determining of a confidence interval from the linearity confidence interval includes:
Determining, based on a measurement parameter within the measurement data, a confidence interval corresponding to the measurement parameter from the linearity confidence interval.
In yet another alternative implementation, the method further includes:

Periodically updating the linearity confidence interval; or
Updating the linearity confidence interval in either of the following situations:

In the measurement data, there is data exceeding the linearity confidence interval;
Process parameters of the gas analysis system are adjusted; and
Hardware of the gas analysis system is replaced.

According to another aspect of the embodiments of the present application, an apparatus for verifying a gas analysis system is provided. The apparatus includes:
A measurement module, configured to perform the following operations:

Testing the gas analysis system using respective cylinder gases with a plurality of standard operating conditions, to determine a plurality of baseline linearity curves of the gas analysis system and a corresponding first confidence interval thereof;
Testing the gas analysis system using simulated operating condition data, to determine a second confidence interval for the plurality of baseline linearity curves, wherein the simulated operating condition data is obtained by simulation of a simulation system on actual operating conditions of an object to be measured;
Testing the gas analysis system using ideal operating condition data of the object to be measured to determine a third confidence interval for the plurality of baseline linearity curves; and
Testing the gas analysis system using first actual operating condition data of the object to be measured, to determine a fourth confidence interval for the plurality of baseline linearity curves; and
A processing module, configured to verify the gas analysis system based on the first confidence interval, the second confidence interval, the third confidence interval, and the fourth confidence interval.

According to another aspect of the embodiments of the present application, an electric device is provided. The electric device includes: a memory, a processor, and a computer program stored on the memory, wherein the processor executes the computer program to implement the steps of the method according to any of the precedent embodiments.
According to yet another aspect of the embodiments of the present application, a computer-readable storage medium is provided. The computer-readable storage medium has a computer program stored thereon, wherein the computer program, when executed by a processor, implements the steps of the method according to any of the precedent embodiments.
According to yet another aspect of the embodiments of the present application, a computer program product is provided, comprising a computer program, wherein the computer program, when executed by a processor, implements the steps of the method according to any of the precedent embodiments.
With the technical solution provided by the embodiments of the present application, it is possible to achieve the beneficial effects including: by verifying the gas analysis system based on the first confidence interval obtained in standard operating conditions, the second confidence interval obtained in simulated operating conditions, the third confidence interval obtained in ideal operating conditions, and the fourth confidence interval obtained in actual operating conditions, it is enabled to determine the validity of data measured by the gas analysis system.

## Brief Description of the Drawings

To explain the technical solutions in the embodiments of the present application more clearly, the following briefly introduces the accompanying drawings used for descriptions of the embodiments.

FIG. 1 is a schematic flowchart of a method for verifying a gas analysis system according to an embodiment of the present application;
FIG. 2 is a schematic structural diagram of an apparatus for verifying a gas analysis system according to an embodiment of the present application;
FIG. 3 is a schematic structural diagram of an electric device according to an embodiment of the present application.

## Detailed Description

The embodiments of the present application are described below in conjunction with the accompanying drawings. It should be understood that the implementations described below in conjunction with the drawings are exemplary descriptions for explaining the technical solutions of the embodiments of the present application and do not constitute limitations on the technical solutions of the embodiments of the present application.

Those skilled in the art will understand that the singular forms "a", "an", "the", and "said" used herein may also encompass plural forms, unless otherwise indicated. It should be further understood that, the terms "comprise" and " comprising" used in the embodiments of the present application mean that the corresponding features can be implemented as the presented features, information, data, steps, operations, elements, and/or components, but do not exclude the implementation of other features, information, data, steps, operations, elements, components, and/or combinations thereof supportable in the technical field. It should be understood that when an element is referred to being "connected" or "coupled" to another element, the element can be directly connected or coupled to another element, or the element can establish a connection relationship with another element through an intermediate element. In addition, the terms "connected" or "coupled" as used herein may include wireless connection or wireless coupling. The term "and/or" as used herein indicates at least one of the items defined by the term. For example, "A and/or B" can be implemented as "A", or as "B", or as "A and B".

To make the objectives, technical solutions, and advantages of the present application apparent, the following further describes the implementations of the present application in detail with reference to the accompanying drawings.

In some applications and fields with high-precision and requiring continuous and precise determination of data validity, typically, it is known that mass concentration (calculated based on volume concentration) * gas flow rate = total emissions. It can be seen that, regardless of being used for process control, for energy efficiency control, or for precise measurement of total amount, volume concentration, or mass concentration, the determination on data validity of volume concentration, mass concentration conversion, and gas flow rate directly affects the final result.

The aforementioned applications and fields include, but are not limited to: continuous precise measurement of total gas volume, precise tracking of volume concentration changes, precise measurement of mass concentration, high-precision process control for precisely tracking and measuring the impact of flow rate, temperature, and pressure on gas processes, energy efficiency control, etc.

Specifically, taking the precision of data for volume concentration and mass concentration as an example. In use of analysis systems (including continuous analysis and/or providing temporary short-term analysis), analytical sensors such as optical sensors based on NDIR (Non-Dispersive Infrared), ultraviolet light sources, or modulated spectrum lasers, or electrochemical sensors are mainly used for volume concentration determination and measurement, which in turn is used to calculate the mass concentration. There is several problems present in this process:

a. Sensor calibration typically uses two-point calibration, i.e., using cylinder gases to calibrate the zero point and span point of the sensor, thereby obtaining a baseline with a fixed slope. Depending on various principles of sensors, the data in actual application with respect to the baseline usually only exhibits good baseline reproducibility or slope close to the baseline within a certain range;
b. The measurement results from sensors merely represent the volume concentration of the gas fed into the sensor, but cannot directly and effectively reflect the actual volume concentration of the sample at the sampling location in the process pipeline. Therefore, the actual volume concentration in the process pipeline might differ significantly from the analysis result;
c. In converting of the volume concentration measured by the sensor into mass flow rate, the influence of gas temperature and pressure on the concentration should be included in a formula for converting volume concentration to mass concentration to become complete and scientific;
d. Analytical sensors rely on processing of sampling and pretreatment equipment on the sample gas. This process can significantly change temperature, humidity, pressure, and flow rate of the sample gas. Changes in these parameters in turn have a huge impact on the volume concentration of the component to be detected in the gas. As such, continuous and dynamic assessment is necessary to relieve the impact on the volume concentration analysis results and to correct the changes;

e. Continuous, uninterrupted, and dynamic correction and calibration is required for analysis result data, based on process changes, environmental changes, and analysis concentration result changes.

Specifically, the descriptions below are made taking the accuracy of gas flow rate data as an example. Flow measurement mainly uses differential pressure flow meters (Pitot tube principle) or mass flow meters. The problems mainly present in measurement lie in:

a. The measurement point of flow rate selected in the process pipeline shall be capable of representing the actual flow rate of the pipeline if the flow rate changes;

b. Process pipeline wear, accumulation of deposits, or modifications may cause impact on the data changes to be corrected;

c. Various installation positions, changes in pipe diameter before and after the installation point, changes in pipe travel direction, and device resistance may cause impact on the data changes to be corrected;

d. Blockage or wear of measurement hardware due to particles or liquids in the process pipeline may cause impact on the actual detection to be corrected.

In summary, in practice, the main reasons resulting in errors in total gas emission results at least include, but are not limited to:

(1) System calibration level, control accuracy errors, sampling and pretreatment control level, installation standardization level, maintenance level, and data processing capability etc. may bring errors into the measured volume concentration, which can also be referred to as volume concentration error.

(2) In converting of system volume concentration into mass concentration, using incomplete calculation formulas (e.g., simply using an empirical formula for converting volume concentration to mass concentration under standard gaseous conditions) may make it impossible to reflect true mass concentration in the process pipeline based on the sensor volume concentration, which can also be referred to as mass concentration error.

(3) Incorrect assessment and calculation of the impact on volume concentration determination caused by changes in the system usage environment, such as changes in ambient temperature and humidity affecting the sensor and system, leading to changes in volume concentration measurement data and impact on the conversion to the true mass concentration in the process pipeline, which can also be referred to as environmental impact error.

(4) Error between process pipeline flow measurement data and the calibration database established based on design parameters, which can also be referred to as flow rate error.

(5) Other influencing factors, such as the execution standard for system automatic calibration, special climate influences, operation and maintenance of process equipment, and changes in fuel or raw material quality may cause data errors or deviations, which can also be referred to as other impact errors.

Due to the existence of the various errors mentioned above, it is hard to determine the accuracy of data measured by the analysis system. In this regard, the present application provides a method, apparatus, electric device, computer-readable storage medium, and computer program product for verifying a gas analysis system, to solve the aforementioned technical problems in the prior art.

The solution of the present application can improve the controllable accuracy and data quality of gas analysis data within control boundaries (corresponding to the linearity confidence interval mentioned above).

The method according to embodiments of the present application may make continuous determination and verification on data quality by establishing boundaries and control procedures. When preset change for application parameters of the system occur, it is possible to provide accurate correction to the data timely. In case where data is determined to be invalid, it is possible to provide effective next-step processing, maintaining the system always at the preset data control accuracy, or providing possibilities for traceability of the next upgrade, modification, or data evaluation. The following mainly summarizes its functions:

a. Establishing a data control boundary and control method for the entire process of the system, accomplishing a dynamic data control database covering the full range including sensors, system components, and process application states, thereby effectively improving the controllable accuracy of data than a single linear baseline calibration system;

b. Enabling comprehensive traceability of system data, providing more parameters for correction on key system data, thereby being capable of effectively tracing where the corrected or calibrated data come from;

c. For those system data jumps occurring in changes of application parameters out of preset ranges, enabling timely and continuous determination of data accuracy and validity;

d. When system data is abnormal but within the system control boundary, timely shifting or extrapolating the data judgment formula to achieve timely and continuous controllable data accuracy;

e. When system data is abnormal and exceeds the system control boundary, providing timely automatic system calibration or supplementing boundaries to establish new calibration baselines and widths, to adapt to continuously controllable data accuracy over a wider control boundary;

f. When system data exceeds the system controllable range, promptly recording invalid data reserved for system alarms and system function upgrades or modifications.

The technical solutions of the embodiments of the present application and the technical effects achieved by the technical solutions of the present application will be described below through the description of some exemplary embodiments. It should be noted that an embodiment in the following embodiments can be referred to, learned from, or combined with another embodiment. Repeated description for same or similar terms, features, and implementation in different embodiments will be omitted.

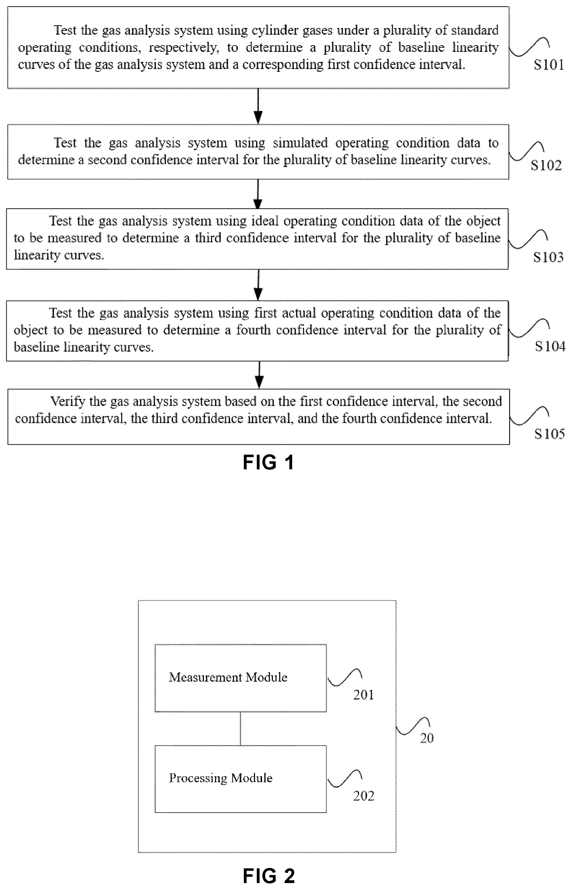

An embodiment of the present application provides a method for verifying a gas analysis system. As shown in FIG. 1, the method includes:

S101: Testing the gas analysis system using respective cylinder gases with a plurality of standard operating conditions, to determine a plurality of baseline linearity curves of the gas analysis system and a corresponding first confidence interval thereof;

S102: Testing the gas analysis system using simulated operating condition data, to determine a second confidence interval for the plurality of baseline linearity curves, wherein the simulated operating condition data is obtained by simulation of a simulation system on actual operating conditions of an object to be measured;

S103: Testing the gas analysis system using ideal operating condition data of the object to be measured, to determine a third confidence interval for the plurality of baseline linearity curves;

S104: Testing the gas analysis system using first actual operating condition data of the object to be measured, to determine a fourth confidence interval for the plurality of baseline linearity curves; and

S105: Verifying the gas analysis system based on the first confidence interval, the second confidence interval, the third confidence interval, and the fourth confidence interval.

It should be noted that in this embodiment, the object to be measured is varied in different application fields or industries. For example, in the shipping industry, the object to be measured may be a marine engine. In fixed process installations, the object to be measured may be a power coal-fired boiler, a steel mill blast furnace, a mine's air return system, etc.

Specifically, in this embodiment, the linearity confidence interval is determined by the gas analysis system under different volume concentrations, ambient temperature and humidity, and process sample gases under different temperature, pressure, flow rate, and humidity conditions. It is the baseline linearity curve obtained under parameters determined by a reference gas source or determinable parameters, and the confidence interval width enveloping these baseline linearity curves, forming a linearity data channel with a certain width. Since it is formed by the continuous accumulation of linearity data channels measured under various operating conditions, it is equivalent to obtaining a control database.

Alternatively, S101 may include: establishing at least five sensor baseline linearity curves and confidence interval widths distributed proportionally between the sensor zero point and span point or close to the main detection range; Determining drift curves per unit time based on sensor design parameters, including at least daily, weekly, and monthly drift curves; forming the data confidence interval width with the baseline linearity curve curvature as the baseline using the baseline linearity curves combined with the time drift curves.

For example, a sensor with a 0-20% vol concentration range should include at least five baseline linearity curves for 0-20%, 40%, 60%, 80%, and 100% of the range, and then, the data confidence interval width may be formed in combination with the sensor drift curve. For example, if the sensor drift is 1% FS/week, the confidence interval width of the baseline curve may be $\pm 1\%$/week.

Then establishing at least five sensor baseline linearity curves and confidence interval widths (corresponding to the first confidence interval) distributed proportionally between the zero point and the maximum span point or close to the main detection range, including all components of the gas analysis system.

For example, the first confidence interval can be obtained by performing the following measurements:

*a*. Measuring the zero point of the system under test under standard operating conditions (temperature, humidity, flow rate) using zero-concentration standard gas;

**b.** Repeating step **(a)** using standard gas with a concentration within the 20% range of the system under test's measurement range;

**c.** Repeating step **(a)** using standard gas with a concentration within the 40% range of the system under test's measurement range;

**d.** Repeating step **(a)** using standard gas with a concentration within the 60% range of the system under test's measurement range;

**e.** Repeating step **(a)** using standard gas with a concentration within the 80% range of the system under test's measurement range.

In an alternative implementation, the first confidence interval can cover the sensor baseline linearity curves and confidence interval width.

It should be noted that since the gas analysis system includes sampling and preparation components, the system's linearity curve is not absolutely covered by the sensor's linearity curve, as components may cause positive or negative influences. Therefore, the system's baseline linearity curves and confidence interval width (corresponding to the first confidence interval) can be determined separately.

It should be noted that when replacing test cylinder gases, the concentration value may change, and a new baseline needs to be re-established after replacement. Each time the test concentration is changed, the first instrument reading should be taken after a time at least three times the response time of the system under test. At least three data points should be read for each test concentration, with the time interval between readings being at least four times the response time. If the process gas is known or determined to contain interfering components before testing, separate testing should be performed and recorded.

In this embodiment, S102 may include: testing the analysis system using different simulated operating conditions configured according to preset parameters (including volume concentration, temperature, pressure, humidity, flow rate, etc.) and test gas concentrations, and obtaining corresponding data.

The preset parameters during testing should refer as much as possible to the maximum parameter range in well-working system, so as to ensure the system test is effective over as wide a range as possible, covering most operating situations.

For example, based on steps a-e above, the second confidence interval can be obtained by performing the following measurements:

**(f)**. Adjust the simulated operating condition temperature parameter to 80% of the standard condition and repeat steps **(a)** to **(e)**;

**(g)**. Adjust the simulated operating condition temperature parameter to 120% of the standard condition and repeat steps **(a)** to **(e)**;

**(h)**. Adjust the simulated operating condition flow rate parameter to 80% of the standard condition and repeat steps **(a)** to **(e)**;

**(i)**. Adjust the simulated operating condition flow rate parameter to 120% of the standard condition and repeat steps **(a)** to **(e)**;

**(j)**. Adjust the simulated operating condition humidity parameter to 80% of the standard condition and repeat steps **(a)** to **(e)**;

**(k)**. Adjust the simulated operating condition humidity parameter to 120% of the standard condition and repeat steps **(a)** to **(e)**;

**(l)**. If components potentially interfering with detection results exist in actual system use, configure them separately and repeat steps **(a)** to **(k)**;

Based on the above test data, obtain and establish a control database for the system's baseline linearity curves and confidence interval widths under different concentration ranges in standard operating conditions.

Testing can also continue based on actual process conditions:

1. Conduct cross-variation tests of variables in preset conditions **(f)** to **(k)** and perform steps **(a)** to **(e)**;
2. Adjust ambient temperature to 0°C and repeat step **(a)**;
3. Adjust ambient temperature to 40°C and repeat step **(a)**;
4. Adjust ambient temperature to 50°C and repeat step **(a)**;
5. Adjust ambient temperature and humidity to 40°C, 30% RH, and repeat step **(a)**;
6. Test according to the actual working location's environmental parameters and repeat step **(a)**.

In the above steps, the following points should be noted:

1) Zero-concentration standard gas and standard test gases of different concentrations should have verifiable quantity and quality;

2) System data recording intervals should be clearly defined; shorter data recording intervals help improve system data quality;

3) For standard test gas tests, standard gases can be obtained from different cylinders or prepared from a single gas concentration using a calibrated dilution system;

4) The concentration ratio in the simulation system needs to be known precisely to avoid failure of linearity testing;

5) Each time test conditions are changed and stabilized, record the first reading after a time at least equal to three times the system response time;

6) When adjusting between steps, data recorded in the first 15 minutes and the last 15 minutes should not be collected for calculation but can be used as reference for experimental analysis data;

7) At least three readings should be taken for each test. The time interval between readings should be at least four times the response time;

8) Negative values during measurement trials should be recorded as such and not forced to zero.

In this embodiment, S 103 may include: On the basis of the process-simulated baseline linearity curves and confidence interval widths, establish a dataset of baseline linearity curves and confidence intervals including changes in environmental parameters (may be corresponding to the third confidence interval).

Specifically, use a test bench or simulation device similar to the actual process (e.g., pilot plant) to simulate the output of actual operating conditions according to preset ideal parameters. Use the system to record and determine that the system's baseline linearity curves and confidence intervals can fully cover the test data, environmental data, and fuel or raw material quality condition data under the test conditions of this stage. If the system recorded data exceeds the set covered by the original baseline linearity curves and confidence intervals, form a new union database covering the ship engine test bench range and the aforementioned ranges.

Specifically, in this embodiment, the following operations can be performed:

* Use the system under test to collect data under preset power change curves;
* Use the system under test to collect data under preset load change curves;
* Use the system under test to collect data under preset power-load cross-change curves.

For example, the third confidence interval can be obtained by performing the following measurements:

* Common power test: Conduct multiple measurements under 85% constant power and load variation range of 10%-100%;
* Cross-variation test: Conduct multiple measurements under power variation range of 10-100% and load variation range of 10%-100%.

Specifically, in this embodiment, the measurable components and measurement conditions in fuel or production raw materials that can be converted into emission gases include the basic conditions during fuel detection and the detection information of the fuel or raw materials. The basic conditions during fuel detection include: sampling environmental conditions, sampling records, sample preparation records, detection environment records, inspection time, inspection batch, and other parameters related to the accuracy and representativeness of the inspection information. Fuel or raw material detection information includes: moisture content, carbon content, low calorific value, net calorific value, simulated distillation value, lower heating value, hydrocarbon composition and content, impurity content, and other parameters related to combustion efficiency.

Parameters related to fuel or raw material consumption include, but are not limited to: total refueling data, total consumption data, real-time consumption data, initial data, silo capacity and design data, pipeline design data, supply system design data, heating device or processing device design data, etc.

Parameters related to the process installation include, but are not limited to: gas system design drawings, installation design parameters, design power parameters, design load parameters, segmented real-time power parameters, segmented real-time load parameters, process pressure data, process temperature parameters, supercharger parameters, maintenance records, etc.

Installation operating parameters include, but are not limited to: usage time, maintenance time, maintenance records, modification records, modification drawings, etc. Environment-related data includes: ambient temperature, ambient humidity, altitude location, climate data, etc. Metering equipment data includes: relevant parameters of collection equipment related to data verification, metering equipment, maintenance records, and usage records.

In an alternative implementation, S105 may include:

* If the fourth confidence interval is entirely located within the union range of the first confidence interval, the second confidence interval, and the third confidence interval, then the gas analysis system passes verification;
* Determine the union range of the first confidence interval, the second confidence interval, and the third confidence interval as the linearity confidence interval.

In this embodiment, if the entirety of the fourth confidence interval determined based on actual operating conditions is covered by, i.e., falling in the combined confidence intervals determined based on standard gas, simulated conditions, and ideal conditions, it indicates that the data obtained by the gas analysis system is accurate. Simultaneously, the combined confidence intervals determined based on standard gas, simulated conditions, and ideal conditions are determined as the linearity confidence interval, to serve as a basis for determining the accuracy of analysis results when using this gas analysis system for gas analysis in the future.

In an alternative implementation, S105 may further include:

* If not entirety of the fourth confidence interval fall in the union range of the first confidence interval, the second confidence interval, and the third confidence interval, then measure the gas analysis system using first operating condition data to determine a fifth confidence interval for the plurality of baseline linearity curves,
* Wherein the first operating condition data is obtained from the simulated operating condition data and second actual operating condition data, and the second actual operating condition data corresponds to the portion of the first actual operating condition data associated with the part of the fourth confidence interval not covered by the union range of the first confidence interval, the second confidence interval, and the third confidence interval;
* Determine the union of the first confidence interval, the second confidence interval, the third confidence interval, the fourth confidence interval, and the fifth confidence interval as the linearity confidence interval.

Specifically, in this embodiment, if the fourth confidence interval determined based on actual operating conditions is not fully covered by the combined confidence intervals determined based on standard gas, simulated conditions, and ideal conditions, it is necessary to update the simulated operating condition data based on the actual operating condition data that exceeds the combined interval and re-determine a new confidence interval (corresponding to the fifth confidence interval above). Simultaneously, the combined confidence intervals determined based on standard gas, simulated conditions, ideal conditions, and actual conditions, along with the newly determined confidence interval, are determined as the linearity confidence interval, to serve as a basis for determining the accuracy of analysis results when using this gas analysis system for gas analysis in the future.

Specifically, in this embodiment, based on data obtained during actual use, if parameters exceeding the existing control database exist, use the simulation system to reproduce the actual operating conditions, repeat the system test in the simulation system, and obtain new system data. Review and update the established data control database by reviewing the system data under actual operating conditions.

For example: The process for updating the test when some parameters in actual operating conditions exceed the existing parameters in the data control database is as follows:

1. Use air or zero-concentration gas to measure the system zero point under simulated actual ship operating conditions (temperature, humidity, flow rate);
2. Repeat the above step using gas with a concentration within the 60% range of the actual operating condition range;
3. Repeat the above step using gas with a concentration within the 80% range of the actual operating condition range;
4. Repeat the above step using gas with a concentration within the 100% range of the actual operating condition range;
5. Repeat the above step using gas with a concentration within the 120% range of the actual operating condition range;

In the above steps, the following points should be noted:

1) Zero-concentration standard gas and standard test gases of different concentrations should have verifiable quantity and quality;
2) System data recording intervals should be clearly defined; shorter data recording intervals help improve system data quality;
3) Standard gases can be obtained from different cylinders or prepared from a single gas concentration using a calibrated dilution system;
4) The concentration ratio in the simulation system needs to be precisely controllable;
5) Each time test conditions are changed and stabilized, record the first reading after a time at least equal to three times the system response time;
6) Data recorded 15 minutes before and after the test should not be collected for calculation;
7) Negative values during measurement trials should be recorded as such and not forced to zero;

8) When replacing calibration standard gas, the concentration value may change, and the impact on the baseline linearity should be checked.

In an alternative implementation, the method further includes:

* Measure third actual operating condition data of the object to be measured using the gas analysis system to obtain measurement data;
* Determine a confidence interval from the linearity confidence interval based on the measurement data;
* Determine invalid data and valid data within the measurement data based on the determined confidence interval.

In an alternative implementation, said determining invalid data and valid data within the measurement data based on the determined confidence interval includes:

* Determining first data within the measurement data that does not exceed the determined confidence interval as the valid data;
* Determining second data within the measurement data that exceeds the determined confidence interval at a first timing or within a first time period as the invalid data.

Specifically, in this embodiment, when measurement data is within the range of the control database, it can be determined as valid data. However, valid data does not mean it is completely correct data; the data still needs to be corrected or calibrated to become data that can be used for output and further application.

In this embodiment, all raw data should be recorded, including current signal (mA) readings of gas sensors, system temperature, pressure, flow rate, and other related sensors. Regardless of whether the system has been corrected, only positive values should not be recorded, nor should negative values be recorded as zero or forced to zero.

The time interval between each reading and recording is the data recording frequency of the system. A faster recording frequency can better reflect changes in the analyzed gas, but it may also cause data jumps or instability. Therefore, this method adopts a dual-frequency recording method. One set of high-speed frequency is determined based on the possible change time of the gas being measured, and the other set of stable frequency is determined based on the system stabilization time and response time. The response time of the gas analysis system is the time from when the gas to be measured is introduced until the first reading appears. The stabilization time is the time from when the gas to be measured is introduced until the reading stabilizes at the actual gas volume concentration.

For example: High-speed frequency data records sensor reading data in the system by seconds or milliseconds, while the stable frequency is determined as 15 seconds or 1 minute based on the system stabilization time or response time; typically, the stable frequency is 3 times the system response time or 1 time the stabilization time.

Alternatively, the second data can also be determined as invalid data if at least one of the following conditions is met:

* Exceeding a first preset value of the measurement range of a sensor in the gas analysis system;
* Detection time is less than the stabilization time of the sensor;
* Detection time is less than the response time of the sensor.

Specifically, in this embodiment, when switching detection conditions is required, the detection time should exceed the stabilization time. For example: When switching from Auxiliary Engine 1 to Auxiliary Engine 2, the instrument needs sufficient time to stabilize; data before reaching a stable state is invalid. This also applies to a single operating condition because the stabilization time is usually longer than the response time. Therefore, data with detection time less than the response time is invalid.

In this embodiment, the first time period can be the time of one data acquisition frequency, or a time longer than 4 times the stable frequency. That is, data exceeding the width of the linearity confidence interval within one data acquisition frequency is invalid. Or, data forming a curve slope that has deviated from all baseline linearity curve slopes in the system control database over a continuous period longer than 4 times the stable frequency, and simultaneously broken through the maximum allowable confidence interval width, is invalid.

In some embodiments, data is recorded as invalid under the following circumstances:

1. Recorded data exceeds 25% or more of the sensor measurement range (corresponding to the first preset value above); for example, the sensor signal is 4-20mA, but a signal below 3mA or above 25mA is recorded, then the data is recorded as invalid.
2. When a single sensor needs to switch detection to more than one sample, data recorded in less than the sensor stabilization time.

3. When a single sensor detects a single sample, data recorded in less than the sensor response time.

4. Sensor data exhibiting abnormal jumps exceeding the confidence interval width within one data frequency due to uncertain reasons not caused by process adjustments (e.g., power fluctuations).

5. Data forming a curve slope that has deviated from all baseline linearity curve slopes in the system control database over a continuous recording period longer than 4 times the stable frequency, and simultaneously broken through the maximum allowable confidence interval width, should be determined as invalid.

6. If any item in the actual measurement data exceeds the data boundary value recorded in the control database and the device's allowable parameter boundary value, all detection data is determined as invalid.

In an alternative implementation, said determining a confidence interval from the linearity confidence interval includes: determining a confidence interval corresponding to a measurement parameter from the linearity confidence interval based on a measurement parameter within the measurement data.

Specifically, in this embodiment, suppose the system has established linearity curves for 0-20% or 0-40% within its 0-10% vol range. Assume the confidence interval for the 0-20% curve is determined to be 1.5-2.0% based on other parameters, and the confidence interval for the 0-40% curve is 1.6-1.9%.

If the sensor volume concentration reading fluctuates between 1.8-2.1% vol, typically the 0-20% range curve should be selected to satisfy the linearity curve covering the measurement data as much as possible. However, when the data exceeds 2% vol volume concentration, the 0-20% range curve will not be able to fully satisfy. But the linearity of the 0-40% curve for the actual reading is worse in controllability compared to the 0-20% curve. Therefore, for readings between 1.9-2%, use the 0-20% curve to determine the confidence interval, while for 2.1% data, use the 0-40% curve to determine the confidence interval.

Another scenario with the same parameters might be: At 1.8% vol concentration, the confidence interval corresponding to the 0-20% curve is 1.9%; while the confidence interval corresponding to the 0-40% curve is 1.7%. In this case, the data confidence interval of the 0-40% curve should be selected. In actual use, the confidence interval will also be determined based on comprehensive data from other sensors to satisfy the most accurate confidence interval possible.

It should be noted that due to their optical characteristics, sensors do not have perfectly consistent linearity. The best linearity of an actual sensor is usually within 30-70% of its range. By establishing multiple linearity curves, denser and higher-precision control lines within the range can be obtained.

In an alternative implementation, the method further includes:

* Periodically updating the linearity confidence interval; or
* Updating the linearity confidence interval in any of the following situations:

 * Process parameters of the gas analysis system are adjusted; and
 * Hardware of the gas analysis system is replaced.

In actual use, situations may arise where system data exceeds the data control database range, usually caused by process adjustments, system updates, etc. In this case, supplementary tests are needed to update the system data control database. Data before updating the data control database should be recorded as invalid data. After updating, the invalid data can be reviewed and verified; if compliant, it can be modified and recorded as valid data.

Due to system hardware replacement, such as: filter element replacement, pipeline length change, replacement of main components, the system will be affected to some extent, mainly manifested as impacts on the temperature, pressure, and flow rate of the gas entering the sensor. The baseline linearity curves in the existing data control database were determined based on the initial state of the system. Therefore, after system hardware replacement, the system's baseline linearity should be reviewed and verified to determine the impact of component replacement on the data control database and perform a data control database update.

In this embodiment, the data control database can also be updated periodically to adapt to actual measurement needs. The period can be set according to the user's actual requirements, which is not limited in this embodiment of the present application.

The linearity confidence interval in the embodiments of the present application is a control database formed by the continuous accumulation of data channels measured under various operating conditions, ultimately forming a union. When measurement data is within the range of the control database, it can be determined as valid data. Valid data is data that can be used for further correction or has controllable accuracy and is located within the confidence interval. By correcting or further calibrating and regressing the data, it becomes data that can be used for output and further application.

Specifically, in this embodiment, system data correction applies to partially controllable errors. The correction method is selected differently based on the content of the error:

1. Correction to Volume Concentration Error

Use the sensor data from the system itself to calculate the difference between the gas sample state (temperature, pressure, flow rate, humidity) in the process pipeline and the state before the gas sensor. Perform data correction in the following steps based on the data differences:

* Calculate the change in gas dew point generated during gas collection and processing.
* Calculate the total amount of condensate based on the gas dew point change.
* Calculate the gas dissolution loss and its error impact on volume concentration based on the total condensate amount and gas solubility.
* Simultaneously, correct for possible mechanical drift, optical drift errors in volume concentration parameters caused by factors such as increased system usage time, based on relevant parameters of metering equipment, maintenance records, and usage records.

2. Correction to Mass Concentration Error

The data obtained by the system is the volume concentration measured by the sensor. When converting to mass concentration, it should be converted to standard state/conditions or to process state conditions.

For example: Given under standard conditions (0 °C, 1013 hPa, dry flue gas with 11% oxygen content.

$$\hat{y}_s = \hat{y} \times \frac{t + 273.15K}{273.15K} \times \frac{1013hPa}{1013hPa + p} \times \frac{100\%}{100\% - h} \times \frac{21\% - o_s}{21\% - o}$$

Where, t is Celsius temperature; p is the difference between sample gas static pressure and standard pressure; h is absolute water vapor content (by volume).

3. Correction to Environmental Influenced Error

The system is installed in the usage environment. Use ambient temperature and humidity to correct the following data:

* Correct the gas temperature before entering the sensor based on ambient temperature.
* Compare and correct the temperature control during sample collection and preparation based on ambient humidity.

4. Correction to Flow Rate Error

For example, use CFD simulation technology combined with engine exhaust system design drawings, measured engine exhaust system structure data, engine design data, engine design power data, engine design load data, engine turbocharger data, engine maintenance records, etc., to establish an engine exhaust vs. power/load model. Use this model to form a correction database and correction function. Use this correction function to correct the measured gas emission flow rate data.

Use climate data to correct data errors caused by sensor displacement due to ship swaying.

For power boilers, coal mines, etc., CFD simulation technology can also be used to establish pipeline aerodynamic simulation diagrams for boilers or mines. Use the power boiler's factory parameters, the mine air return system fan design parameters combined with CFD and usage conditions to establish an aerodynamic model, matching the actual process pipeline flow rate changes under different operating conditions, boiler output conditions, and mine fan speeds. Based on regression comparison between actual test data and CFD simulation data, determine the error situation and perform continuous dynamic correction.

It should be noted that in this embodiment, data calibration is applied in two aspects: one is to form calibration functions during the process of establishing the system control database; the other is to calibrate the data obtained during the actual measurement process after the control database is established.

The calibration function is effective when data during its establishment process falls within the valid calibration range. The valid calibration range refers to the measurement value being within the calibration range of the calibration system from zero to maximum under standard conditions and the width generated by the determined confidence interval. For data outside the valid calibration range, if it exhibits a shift parallel to the baseline linearity curve, testing over a larger range can be used to implement extrapolated calibration curves or establish new calibration curves and functions.

After establishing the calibration function, conduct variability tests on the data to ensure the calibration function remains continuously effective. Continuous data calibration achieves the following control functions:

First, for data at the junction of two baseline linearity curves:
Determine the baseline linearity ultimately used for data regression based on the data operation trend, always ensuring the data is within a higher controllable accuracy range.

For example: If the system data control database includes baseline linearity curves for 0-20% range and 0-40% range, and the actual measured data is 18% to 22% showing an upward trend, the slope of the change curve can be compared with the slopes of the two baseline linearity curves. The one with the smaller slope difference is used as the baseline linearity for data comparison, control, and calibration judgment.

Second, for fluctuating data temporarily exceeding control database range:
For fluctuating data exceeding one data recording frequency but less than the system response time range, use the baseline linearity and confidence interval width surrounding the data's location in the control database to determine the data, confirming whether it is normal. If abnormal, record as invalid data; if normal, then determine the corresponding process data again to determine its normality. If the process data is determined abnormal, record as invalid data; if the process data is determined normal, it belongs to data jumps caused by uncertainty, determined and recorded as invalid jump data. Invalid data and invalid jump data are crucial for the continuous operation status of the system and system function upgrades.

Third, for continuously occurring data anomalies exceeding the data control database range:
When this occurs in actual use, data should not be calibrated directly. First, determine the data.

It usually presents a regular trend or an irregular trend. Use the calibration function of the data control database to determine the data. If it presents a regular upward or downward trend, further check if the surrounding baseline linearity and confidence interval are usable. If the rise or fall presents a parallel relationship with the baseline linearity, the system automatic calibration function can be activated, using zero and concentration-close reference gases to execute the system gas calibration procedure, thereby establishing a new calibration curve and function for extrapolation or expanding the control database boundary (usually this situation is caused by significant instrument drift, which can often be regressed to the valid interval by calibrating zero and span). If it presents an inability to calibrate or still does not match the baseline linearity after calibration, it can be determined as a system failure, and simultaneously recorded as invalid data. If the data presents an irregular trend, it should be determined as invalid data, and relevant matching data recorded for later data analysis and fault analysis.

In the above embodiments, system data calibration, on the one hand, uses the baseline linearity and width established based on system tests and experiments; on the other hand, it can introduce more data and models to establish a larger range data calibration control database. Depending on the content of the data needing calibration, the data range used for calibration differs, as follows:
First, Calibration data range for system volume concentration data

1. Baseline linearity curves and confidence intervals of sensors and the gas analysis system: Verify whether the measured volume concentration parameters conform.
2. Drift parameters of system equipment: Used to shift the baseline linearity when system data drifts based on usage time.
3. Detection information related to fuel or raw materials converted into emission gases: Calculate possible maximum limit data for post-combustion volume concentration.
4. Process installation design parameters: Volume concentration maximum limit data under different operating conditions.
5. System maintenance records: Used to determine the working state of the gas analysis system and sensors during automatic calibration or calibration, further determining the curve to be used.
6. Installation usage time and maintenance records: Used to determine the linearity of increasing emission volume concentration caused over time.

The effective calibration range for gas volume concentration can be established through the above process.

Second, Calibration data range for system mass concentration data

Use fuel or raw material inspection data to directly measure and calculate the mass concentration of the target gas after use. This data can be used as a reference for the mass concentration of the current batch of fuel. Due to fuel inconsistency and detection errors, this data is generally used for establishing calibration curves, not for direct mass concentration verification.

Third, Calibration data range for system flow rate data

Use installation power data, load data, operating-related temperature, pressure, and flow rate data to establish exhaust calibration curves and functions. In actual operating conditions, establish a flow rate data calibration model by creating a CFD model of the installation detection location and using known data at input points and possible data relationships within the model. Since flow rate data has many influencing parameters, the data control database here has a relatively large confidence interval width. It can accumulate data continuously with system usage, gradually narrowing the confidence interval width. The initial data control database is mainly used to determine whether the data is within a valid confidence interval.

Fourth, Data used for calibrating total system emission data

Fuel consumption can be obtained relatively accurately or calibration function curves established through fuel total refueling data, fuel total consumption data, fuel real-time consumption data, fuel initial data, fuel silo design data, fuel pipeline design data, fuel supply system design data, fuel heating device or processing device design data, etc.

The fuel carbon conversion factor has significant uncertainty and is greatly affected by fuel quality. Therefore, most system emission equivalent calibration data is used to determine the maximum limit for system total emission data.

An embodiment of the present application provides an apparatus for verifying a gas analysis system. As shown in FIG. 2, the gas analysis system verification apparatus 20 may include: a measurement module 201 and a processing module 202. The measurement module 201 is configured to perform the following operations:

* Testing the gas analysis system using respective cylinder gases with a plurality of standard operating conditions to determine a plurality of baseline linearity curves of the gas analysis system and a corresponding first confidence interval;
* Testing the gas analysis system using simulated operating condition data, to determine a second confidence interval for the plurality of baseline linearity curves, wherein the simulated operating condition data is obtained by simulation of a simulation system on actual operating conditions of an object to be measured;
* Testing the gas analysis system using ideal operating condition data of the object to be measured, to determine a third confidence interval for the plurality of baseline linearity curves;
* Testing the gas analysis system using first actual operating condition data of the object to be measured, to determine a fourth confidence interval for the plurality of baseline linearity curves;

The processing module 202 is configured to verify the gas analysis system based on the first confidence interval, the second confidence interval, the third confidence interval, and the fourth confidence interval.

Further, the processing module 202 is specifically configured to:

* If the fourth confidence interval is entirely located within the union range of the first confidence interval, the second confidence interval, and the third confidence interval, then the gas analysis system passes verification;
* Determine the union range of the first confidence interval, the second confidence interval, and the third confidence interval as a linearity confidence interval.

In another alternative implementation, the measurement module 201 is further configured to:

* If the entirety of the fourth confidence interval falls in the union range of the first confidence interval, the second confidence interval, and the third confidence interval, measure the gas analysis system using first operating condition data to determine a fifth confidence interval for the plurality of baseline linearity curves;
* Wherein the first operating condition data is obtained from the simulated operating condition data and second actual operating condition data, and the second actual operating condition data is set as a portion of the first actual operating condition data corresponding to the remaining of the fourth confidence interval exposed out of the union range of the first confidence interval, the second confidence interval, and the third confidence interval;

The processing module 202 is specifically configured to: determine the union of the first confidence interval, the second confidence interval, the third confidence interval, the fourth confidence interval, and the fifth confidence interval as the linearity confidence interval.

In yet another alternative implementation, the measurement module 201 is further configured to: measure third actual operating condition data of the object to be measured using the gas analysis system to obtain measurement data;

The processing module 202 is further configured to: determine a confidence interval from the linearity confidence interval based on the measurement data; determine invalid data and valid data within the measurement data based on the determined confidence interval.

In yet another alternative implementation, the processing module 202, in determining invalid data and valid data within the measurement data based on the determined confidence interval, is specifically configured to: determine first data within the measurement data that does not exceed the determined confidence interval as the valid data; determine second data within the measurement data that exceeds the determined confidence interval at a first timing or within a first time period as the invalid data.

In yet another alternative implementation, the processing module 202, when determining a confidence interval from the linearity confidence interval, is specifically configured to: determine, based on a measurement parameter within the measurement data, a confidence interval corresponding to the measurement parameter from the linearity confidence interval.

In yet another alternative implementation, the processing module 202 is further configured to:

* Periodically update the linearity confidence interval; or,
* Update the linearity confidence interval in any of the following situations:

  * In the measurement data, there is data exceeding the linearity confidence interval;
  * Process parameters of the gas analysis system are adjusted; and
  * Hardware of the gas analysis system is replaced.

The apparatus of the embodiments of the present application can execute the method provided by the embodiments of the present application. The implementation principles are similar. The actions executed by each module in the apparatus of the embodiments of the present application correspond to the steps in the method of the embodiments of the present application. For a detailed functional description of each module in the apparatus, please refer to the description in the corresponding method shown above, which will not be repeated here.

An embodiment of the present application provides an electric device, including a memory, a processor, and a computer program stored on the memory. The processor executes the computer program to implement the steps of the method for verifying a gas analysis system. Compared with the related art, it can be realized that the provided method for verifying a gas analysis system enables the determination of the validity of data measured by said gas analysis system.

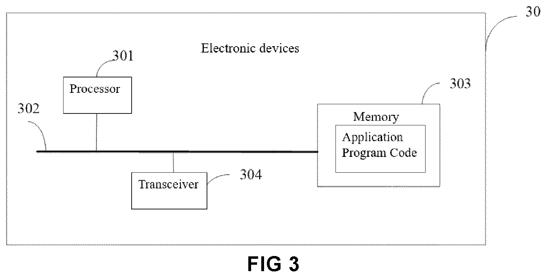

An alternative embodiment provides an electric device. As shown in FIG. 3, the electric device 30 includes: a processor 301 and a memory 303. The processor 301 and the memory 303 are connected, such as via a bus 302. Alternatively, the electric device 30 may further include a transceiver 304. The transceiver 304 can be used for data interaction between this electric device and other electric devices, such as data sending and/or receiving. It should be noted that in practical applications, the number of transceivers 304 is not limited to one, and the structure of the electric device 30 does not constitute a limitation on the embodiments of the present application.

The processor 301 may be a CPU (Central Processing Unit), a general-purpose processor, a DSP (Digital Signal Processor), an ASIC (Application Specific Integrated Circuit), an FPGA (Field Programmable Gate Array), or other programmable logic devices, transistor logic devices, hardware components, or any combination thereof. It can implement or execute various exemplary logic blocks, modules, and circuits described in conjunction with the content disclosed in this application. The processor 301 may also be a combination for computing functions, such as a combination of one or more microprocessors, a combination of a DSP and a microprocessor, etc.

The bus 302 may include a path for transmitting information between the aforementioned components. The bus 302 may be a PCI (Peripheral Component Interconnect) bus, an EISA (Extended Industry Standard Architecture) bus, etc. The bus 302 can be divided into an address bus, a data bus, a control bus, etc. For ease of representation, only one thick line is used in FIG. 3, but it does not mean that there is only one bus or one type of bus.

The memory 303 may be a ROM (Read-Only Memory) or other types of static storage devices capable of storing static information and instructions, a RAM (Random Access Memory) or other types of dynamic storage devices capable of storing information and instructions, or an EEPROM (Electrically Erasable Programmable Read Only Memory), a CD-ROM (Compact Disc Read Only Memory), other optical disc storage (including compact discs, laser discs, optical discs, digital versatile discs, Blu-ray discs, etc.), magnetic disk storage media, other magnetic storage devices, or any other medium that can be used to carry or store computer programs and can be read by a computer, which is not limited herein.

The memory 303 is used to store the computer program for executing the embodiments of the present application and is controlled by the processor 301 to execute. The processor 301 is used to execute the computer program stored in the memory 303 to implement the steps shown in the foregoing method embodiments.

An embodiment of the present application provides a computer-readable storage medium. The computer-readable storage medium has a computer program stored thereon. The computer program, when executed by a processor, can implement the steps and corresponding content of the foregoing method embodiments.

An embodiment of the present application also provides a computer program product, comprising a computer program. The computer program, when executed by a processor, can implement the steps and corresponding content of the foregoing method embodiments.

The terms "first", "second", "third", "fourth", "1th", "2nd", etc. (if present) in the specification and claims of this application and the above drawings are used to distinguish similar objects and do not necessarily state a specific sequence or chronological order. It should be understood that the data used in this way may be interchanged under appropriate circumstances so that the embodiments of the present application described herein can be implemented in sequences other than those illustrated or described in the text.

It should be understood that although the flowcharts in the embodiments of the present application indicate various operation steps through arrows, the implementation order of these steps is not limited by the order indicated by the arrows. Unless explicitly stated herein, in some implementation scenarios of the embodiments of the present application, the implementation steps in each flowchart can be executed in other orders as needed. In addition, some or all of the steps in each flowchart may include multiple sub-steps or multiple stages based on actual implementation scenarios. Some or all of these sub-steps or stages may be executed at the same time, and each sub-step or stage may also be executed at different times. In scenarios where execution times are different, the execution order of these sub-steps or stages can be flexibly configured as needed, which is not limited in the embodiments of the present application.

The above descriptions are only alternative implementations of some implementation scenarios of the present application. It should be pointed out that for those of ordinary skill in the art, without departing from the technical concept of the solution of the present application, other similar implementation means based on the technical idea of the present application also fall within the protection scope of the embodiments of the present application.

## Claims

1. A method for verifying a gas analysis system, **characterized in that**, the method includes:

   testing the gas analysis system using respective cylinder gases with a plurality of standard operating conditions, to determine a plurality of baseline linearity curves of the gas analysis system and a corresponding first confidence interval thereof;

   testing the gas analysis system using simulated operating condition data, to determine a second confidence interval for the plurality of baseline linearity curves, wherein the simulated operating condition data is obtained by simulation of a simulation system on actual operating conditions of an object to be measured;

   testing the gas analysis system using ideal operating condition data of the object to be measured, to determine a third confidence interval for the plurality of baseline linearity curves;

   testing the gas analysis system using first actual operating condition data of the object to be measured, to determine a fourth confidence interval for the plurality of baseline linearity curves; and

   verifying the gas analysis system based on the first confidence interval, the second confidence interval, the third confidence interval, and the fourth confidence interval.

2. The method according to claim 1, **characterized in that**, the verifying of the gas analysis system based on the first confidence interval, the second confidence interval, the third confidence interval, and the fourth confidence interval includes:

   if entirety of the fourth confidence interval falls in a union range of the first confidence interval, the second confidence interval, and the third confidence interval, the gas analysis system passing verification; and

   determining the union range of the first confidence interval, the second confidence interval, and the third confidence interval as a linearity confidence interval.

3. The method according to claim 2, **characterized in that**, the verifying of the gas analysis system based on the first confidence interval, the second confidence interval, the third confidence interval, and the fourth confidence interval further includes:

   if not entirety of the fourth confidence interval falls in the union range of the first confidence interval, the second confidence interval, and the third confidence interval, measuring the gas analysis system using first operating condition data to determine a fifth confidence interval for the plurality of baseline linearity curves;

   wherein the first operating condition data is obtained from the simulated operating condition data and second actual operating condition data, and the second actual operating condition data is set as a portion of the first actual operating condition data corresponding to the remaining of the fourth confidence interval exposed out of the union range of the first confidence interval, the second confidence interval, and the third confidence interval; and

   determining the union of the first confidence interval, the second confidence interval, the third confidence interval, the fourth confidence interval, and the fifth confidence interval as the linearity confidence interval.

4. The method according to claim 2 or 3, **characterized in that**, the method further includes:

measuring third actual operating condition data of the object to be measured using the gas analysis system to obtain measurement data;

determining a confidence interval from the linearity confidence interval based on the measurement data; and

determining invalid data and valid data within the measurement data based on the determined confidence interval.

5. The method according to claim 4, **characterized in that**, the determining of invalid data and valid data within the measurement data based on the determined confidence interval includes:

determining first data within the measurement data that does not exceed the determined confidence interval, as the valid data; and

determining second data within the measurement data that exceeds the determined confidence interval at a first timing or within a first time period as the invalid data.

6. The method according to claim 4, **characterized in that**, the determining of a confidence interval from the linearity confidence interval includes:
determining, based on a measurement parameter within the measurement data, a confidence interval corresponding to the measurement parameter from the linearity confidence interval.

7. The method according to claim 4, **characterized in that**, the method further includes:

periodically updating the linearity confidence interval; or

updating the linearity confidence interval in any of the following situations:

process parameters of the gas analysis system are adjusted; and

hardware of the gas analysis system is replaced.

8. An apparatus for verifying a gas analysis system, **characterized by** comprising:

a measurement module, configured to perform the following operations:

testing the gas analysis system using respective cylinder gases with a plurality of standard operating conditions to determine a plurality of baseline linearity curves of the gas analysis system and a corresponding first confidence interval thereof;

testing the gas analysis system using simulated operating condition data, to determine a second confidence interval for the plurality of baseline linearity curves, wherein the simulated operating condition data is obtained by simulation of a simulation system on actual operating conditions of an object to be measured;

testing the gas analysis system using ideal operating condition data of the object to be measured, to determine a third confidence interval for the plurality of baseline linearity curves;

testing the gas analysis system using first actual operating condition data of the object to be measured, to determine a fourth confidence interval for the plurality of baseline linearity curves; and

a processing module, configured to verify the gas analysis system based on the first confidence interval, the second confidence interval, the third confidence interval, and the fourth confidence interval.

9. An electric device, comprising a memory, a processor, and a computer program stored on the memory, **characterized in that** the processor executes the computer program to implement the steps of the method according to any one of claims 1 to 7.

10. A computer-readable storage medium, having a computer program stored thereon, **characterized in that** the computer program, when executed by a processor, implements the steps of the method according to any one of claims 1 to 7.

Test the gas analysis system using cylinder gases under a plurality of standard operating conditions, respectively, to determine a plurality of baseline linearity curves of the gas analysis system and a corresponding first confidence interval. S101

Test the gas analysis system using simulated operating condition data to determine a second confidence interval for the plurality of baseline linearity curves. S102

Test the gas analysis system using ideal operating condition data of the object to be measured to determine a third confidence interval for the plurality of baseline linearity curves. S103

Test the gas analysis system using first actual operating condition data of the object to be measured to determine a fourth confidence interval for the plurality of baseline linearity curves. S104

Verify the gas analysis system based on the first confidence interval, the second confidence interval, the third confidence interval, and the fourth confidence interval. S105

**FIG 1**

Measurement Module 201

Processing Module 202

20

**FIG 2**

Electronic devices

301

Processor

302

303

Memory

Application
Program Code

Transceiver    304

30

**FIG 3**

## INTERNATIONAL SEARCH REPORT

| International application No. |
|---|
| **PCT/CN2024/115173** |

### A. CLASSIFICATION OF SUBJECT MATTER

G01N33/00(2006.01)i; G01D18/00(2006.01)i; G01D21/02(2006.01)i

According to International Patent Classification (IPC) or to both national classification and IPC

### B. FIELDS SEARCHED

Minimum documentation searched (classification system followed by classification symbols)

IPC:G01N G01D

Documentation searched other than minimum documentation to the extent that such documents are included in the fields searched

Electronic data base consulted during the international search (name of data base and, where practicable, search terms used)

CNTXT, ENTXTC, VEN, CNKI, Web of Science: 置信区间, 估计区间, 线性, 范围, 验证, 校准, 校正, 标定, 基准, 标准, 理想, 模拟, 实际, 气体, confidence interval?, fiducial interval?, range, verif+, standard, simulat+, gas??

### C. DOCUMENTS CONSIDERED TO BE RELEVANT

| Category* | Citation of document, with indication, where appropriate, of the relevant passages | Relevant to claim No. |
|---|---|---|
| PX | CN 116879513 A (CHINA CARBON IMMORTAL TESTING (BEIJING) TECHNOLOGY CO., LTD.) 13 October 2023 (2023-10-13)<br>entire document | 1-10 |
| PX | CN 116878559 A (CHINA STANDARD INSPECTION CO., LTD.) 13 October 2023 (2023-10-13)<br>entire document | 1-10 |
| A | CN 113219130 A (CHINA AGRICULTURAL UNIVERSITY et al.) 06 August 2021 (2021-08-06)<br>description, paragraphs 5-52 | 1-10 |
| A | CN 114660241 A (NATIONAL INSTITUTE OF METROLOGY, CHINA et al.) 24 June 2022 (2022-06-24)<br>entire document | 1-10 |
| A | US 2019137465 A1 (HORIBA, LTD.) 09 May 2019 (2019-05-09)<br>entire document | 1-10 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **30 October 2024** | **20 November 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2024/115173**

| Patent document cited in search report | | | Publication date (day/month/year) | Patent family member(s) | | | Publication date (day/month/year) |
|---|---|---|---|---|---|---|---|
| CN | 116879513 | A | 13 October 2023 | CN | 116879513 | B | 14 November 2023 |
| CN | 116878559 | A | 13 October 2023 | CN | 116878559 | B | 10 November 2023 |
| CN | 113219130 | A | 06 August 2021 | CN | CN113219130 | B | 02 August 2022 |
| CN | 114660241 | A | 24 June 2022 | | None | | |
| US | 2019137465 | A1 | 09 May 2019 | US | 10955398 | B2 | 23 March 2021 |
| | | | | CN | 109752485 | A | 14 May 2019 |
| | | | | CN | 109752485 | B | 21 February 2023 |
| | | | | EP | 3480593 | A2 | 08 May 2019 |
| | | | | EP | 3480593 | A3 | 11 September 2019 |
| | | | | EP | 3480593 | B1 | 30 December 2020 |
| | | | | JP | 2019086371 | A | 06 June 2019 |
| | | | | JP | 6985893 | B2 | 22 December 2021 |

Form PCT/ISA/210 (patent family annex) (July 2022)